# EUROPÄISCHE PATENTANMELDUNG

(11) **EP 1 990 008 A1**
(43) Veröffentlichungstag der Anmeldung: **12.11.2008**
(21) Anmeldenummer: 08007805.8
(22) Anmeldetag: 23.04.2008
(51) Int. Cl.: A61B 6/12, A61B 19/00

(54) **Röntgendiagnostikeinrichtung mit einer Vielzahl kodierter Marken**

(30) Priorität: 05.05.2007 DE 102007021185
(71) Anmelder: Ziehm Imaging GmbH, 90451 Nürnberg (DE)
(72) Erfinder: Dehler, Jürgen, 91301 Forchheim (DE)

(57) **Zusammenfassung**

Die Erfindung betrifft eine Röntgendiagnostikeinrichtung, die auf den Oberflächen von Einrichtungsteilen eine Vielzahl von codierten 2D und 3D-Marken aufweist, deren jeweilige Lage und Codierung wenigstens für einen Teil der codierten Marken von einem Lageerfassungssystem erfaßbar ist. Jeder codierten Marke ist ein Referenzpunkt zugeordnet, dessen Lage auf der Oberfläche der Einrichtungsteile bekannt ist, so daß durch die Lagebestimmung der einzelnen Referenzpunkte mittels des Lageerfassungssystems die Lage der Einrichtungsteile im Raum aus dem einhüllenden Referenzpunktnetz errechenbar ist. Die Marken können beispielsweise durch einen Datamatrix-Code (DMC) repräsentiert werden.

## Beschreibung

Medizinische Eingriffe an lebenden Objekten werden zunehmend mittels Navigationsunterstützung durchgeführt. Darunter versteht man die mittels eines Lageerfassungssystems unterstützte Führung eines Instrumentes relativ zu einem in Behandlung stehenden Gewebebereichs des Objektes. Von besonderem Interesse ist die Navigation in Bereichen,-die sich einer optischen Kontrolle des Operateurs entziehen, weil das Instrument beispielsweise in das Innere des Objektes eingeführt wurde. Hierzu wird die Führung des Instrumentes, beispielsweise eines Katheders, in einem virtuellen 3D-Volumen vorgenommen, welches mittels eines bildgebenden Verfahrens vor oder während der Operation erzeugt wurde. Eine häufige Anwendung ist, mit Hilfe einer Röntgendiagnostikeinrichtung eine Reihe von 2D-Projektionsaufnahmen bekannter Projektionsgeometrie zu erzeugen und aus diesen 2D-Aufnahmen einen 3D-Volumendatensatz zu erzeugen. Der Volumendatensatz wird an ein Navigationssystem übergeben, das über ein Lageerfassungssystem für von diesem erfaßbaren Marken verfügt. Um eine Navigation mit hoher Genauigkeit möglich zu machen, wird das Koordinatensystem des Lageerfassungssystem mit dem Koordinatensystem des 3D-Volumendatensatzes abgeglichen. Dieser Vorgang wird üblicherweise "Registrierung" genannt.

Aus der Patentliteratur sind Röntgendiagnostikeinrichtungen bekannt, bei denen Einrichtungsteile mit von einem Lageerfassungssystem erfaßbaren Marken versehen sind.

Aus der deutschen Patentschrift DE 102 06 193 C1 ist bekannt, eine Röntgendiagnostikeinrichtung mit einer Markenanordnung in Form eines zweidimensionalen Barcodes zu verwenden, wobei keine Zuordnung zwischen der codierten Information und der Lage des Barcodes auf dem Gehäuse in einem gerätefesten Koordinatensystem vorgesehen ist.

Aus der DE 102 15 808 B4 ist ein Röntgengerät bekannt, das an der Halterung für den C-Bogen eine Markenanordnung trägt, die mittels eines Lageerfassungssystems erfaßbar ist, wodurch die Lage und Position des Röntgengerätes im Raum bestimmbar ist.

Aus den deutschen Patentschriften DE 103 60 025 B4 und DE 101 39 329 B4 sind Röntgendiagnostikeinrichtungen bekannt, bei denen der Röntgenstrahlenempfänger eine Markenanordnung trägt.

Aus der deutschen Offenlegungsschrift DE 103 52 556 A1 eine codierte Markenanordnung zur Identifizierung und Positionierung von Patienten bekannt.

Aus dem US-Patentdokument US 4 649 504 A ist ein optisches Verfahren zur Bestimmung der Position und der Orientierung eines Festkörpers mit Marken bekannt.

Aus der WO 2006/130 012 A1 ist in Verfahren zur Vortransformation und zur Linearisierung einer Pixelmatrix bekannt, um eine nachfolgende automatische Texterkennung (OCR) zu ermöglichen.

Allen bekannten Röntgendiagnostikeinrichtungen mit Markenanordnungen auf der Oberfläche von Einrichtungsteilen ist gemeinsam, daß sie jeweils nur wenige Marken im Erfassungsraumwinkel des verwendeten Lageerfassungssystems aufweisen und bei ungünstiger Positionierung des Lageerfassungssystems bezüglich der Röntgendiagnostikeinrichtung eine geringe Anzahl auswertbarer Marken verbleibt, was die Genauigkeit der Lagebestimmung einschränkt.

Aufgabe der Erfindung ist es, bei einer vorgegebenen Orientierung zwischen einer Röntgendiagnostikeinrichtung und einem Lageerfassungssystem die Genauigkeit der Positionsbestimmung von Einrichtungsteilen der Röntgendiagnostikeinrichtung gegenüber den bei bekannten Röntgendiagnostikeinrichtungen verwendeten Markenanordnungen zu verbessern.

Die Aufgabe der Erfindung wird dadurch gelöst, daß eine große Zahl von erfaßbaren und unterscheidbaren Marken auf der gesamten Oberfläche der Röntgendiagnostikeinrichtung verteilt ist, wobei jede kodierte Markenanordnung einen Referenzpunkt auf der Oberfläche und einen mit diesem verbundenen Code enthält, der eine Zuordnung des Referenzpunktes zu dem vorbestimmten Ort auf der Oberfläche der Röntgendiagnostikeinrichtung erlaubt. Die Erfindung wird an Hand der Abbildungen näher erläutert.

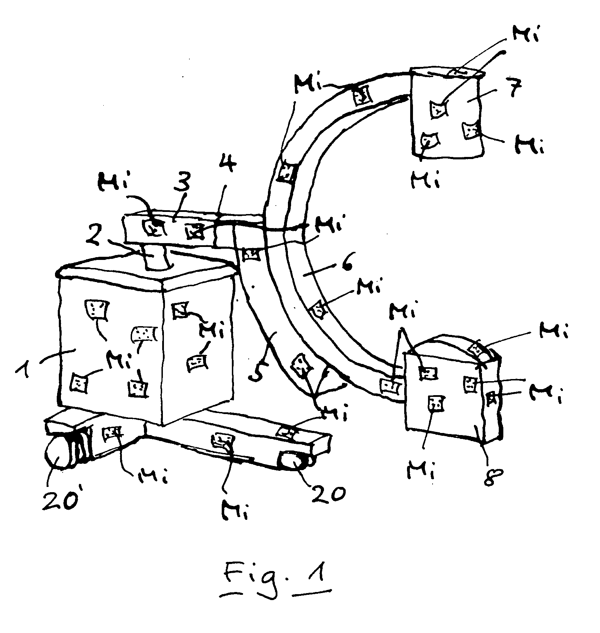

In Fig. 1 ist eine mobile Röntgendiagnostikeinrichtung mit einem auf Rollen (20, 20') längs des Fußbodens verschiebbaren Gerätewagen (1) dargestellt, der einen mehrfach verstellbaren C-Bogen (6) trägt. Die Röntgenstrahlenquelle (8) und der Röntgenstrahlenempfänger (7) sind an den Enden eines C-Bogens (6), der längs seines Umfanges in einer C-Bogenhalterung (5) um einen Mittelpunkt verschieblich gelagert ist, angeordnet. Die C-Bogenhalterung (5) ist an dem Gerätewagen (1) mehrfach verstellbar angeordnet. Die C-Bogenhalterung (5) ist mit einem Schwenklager (4) an einer horizontal verschiebbaren Horizontalführung (3) um eine horizontale Achse schwenkbar gelagert. Die Horizontalführung (3) ist an einer Säule (2) höhenverstellbar und um die senkrechte Achse der Säule (2) drehbar gelagert.

Auf der gesamten Oberfläche der Gehäuse der Einrichtungsteile (1, 3, 5, 6, 7, 8) sowie auf anderen Oberflächen der Röntgendiagnostikeinrichtung ist eine Vielzahl von codierten Markenanordnungen (Mi) verteilt, von denen in der Fig. 1 nur einige wenige dargestellt sind. Die codierten Markenanordnungen (Mi) sind von einem nicht dargestellten Lageerfassungssystem erfaßbar.

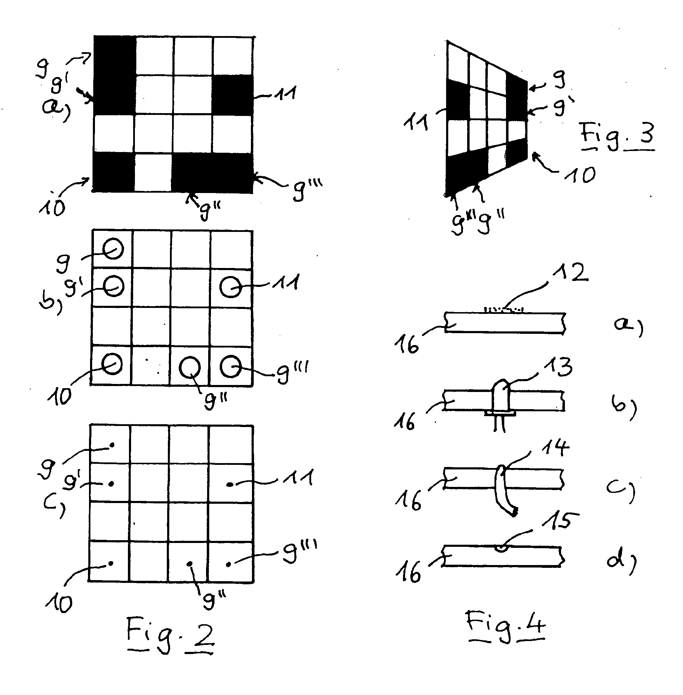

In Fig. 2 ist eine Markenanordnung (Mi) beispielhaft in Form eines zweidimensionalen Matrixcodes dargestellt. Die 2D-Markenanordnung enthält eine Referenzmarke (10) und Positionsmarken (9, 9', 9", 9"') sowie eine Kodiermarke (11) in einem 9 Felder zählenden Kodierbereich. Die Marken (9, 10, 11) sind an einem Raster ausgerichtet und in Fig. 2a in Form von Quadraten ausgeführt. In Fig. 2b sind die Marken (9, 10, 11) kreisförmig und in Fig. 2c punktförmig dargestellt. Die Marken (9, 10, 11) können, wie in Fig. 4a dargestellt, auf der Oberfläche des Gehäuses (16) durch Drucken oder Kleben als Autoreflexionsmarke (12) aufgebracht sein. Die Marken (9, 10, 11) können auch als Lichtquellen ausgebildet sein, beispielsweise als Leuchtdiodenmarke (LED-Marke, Fig. 4b) oder als Lichtleitfasermarke (14, Fig. 4c), die mit einer Mikrolinse abgeschlossen ist. Ferner ist vorgesehen, eine Marke (9, 10, 11) in Form einer Antastmulde (15, Fig. 4d) auszuführen, die mittels eines von dem Lageerfassungssystem erfaßbaren Pointers antastbar ist.

Das Verfahren zum Lesen des Codes und zur Bestimmung der Position der Referenzmarke geht bei Verwendung eines Lageerfassungssystem mit einer Kamera oder mit einer Stereokamera davon aus, daß zunächst an einem aufgenommenen Bild mit den Markenanordnungen (Mi) diese einzeln einer Vortransformation unterzogen werden, wodurch die Abbilder der Markenanordnungen (Mi) auf geneigten oder gekrümmten Flächen in eine Darstellung einer orthogonal aufgenommenen ebenen Markenanordnung (Mi) transformiert wird. Nach durchgeführter Vortransformation, bei der der Ort der Referenzmarke (10) im Gesamtbild erhalten bleibt, wird der Code mit einem bekannten hinterlegten Schlüssel ermittelt. Für die entschlüsselten Codes der Markenanordnungen (Mi) sind in einer look-up-table (LUT) eines Auswerterechners die Positionen der Referenzmarken (10) in einem Koordinatensystem der Röntgendiagnostikeinrichtung oder der Einrichtungsteile (1, 3, 5, 6, 7, 8) hinterlegt. In Fig. 3 ist als Beispiel eine schräg zur ebenen Fläche der Markenanordnung aus Fig. 2a aufgenommene Markenanordnung dargestellt.

Der aus den Meßwerten des Lageerfassungssystems ermittelbare Code ist mit der Position eines definierten Referenzpunktes auf der Oberfläche eines Einrichtungsteils der Röntgendiagnostikeinrichtung eindeutig verknüpft.

Bei den Markenanordnungen Mi kann es sich um einen ebenen 2D-Code oder um einen 3D-Code handeln, der aus einem auf einer gekrümmten Fläche abgewickelten 2D-Code besteht. Als Codes können beispielsweise Strichcodes oder Datamatrix-Codes (DMC) zum Einsatz kommen. Der zu einer bestimmten Markenanordnung (Mi) gehörige Referenzpunkt auf der Oberfläche kann durch Konvention ein Teil des Codes selbst sein oder er ist als Kreis, Quadrat, Fadenkreuz oder dergleichen geometrische Figur im Bereich der Markenanordnung (Mi) festgelegt.

Es ist im Rahmen der Erfindung vorgesehen, die Markenanordnungen (Mi) auf der Oberfläche der Röntgendiagnostikeinrichtung lückenlos nebeneinander anzuordnen.

Die Auswertung der Meßdaten des Lageerfassungssystems erlaubt die Rekonstruktion eines Referenzpunktnetzes auf der Oberfläche und, da die Positionen der Referenzpunkte auf der Oberfläche bekannt sind, die Ermittlung der Lage und Orientierung der Einrichtungsteile im Raum bezüglich des Koordinatensystems des Lageerfassungssystems. Insbesondere ist die Lage und Positionierung jedes einzelnen Einrichtungsteils und dadurch die relative Orientierung der Einrichtungsteile untereinander ermittelbar, was zur Ermittlung der Röntgenprojektionsgeometrien verwendbar ist.

### Bezugszeichenliste:

- 1: Gerätewagen
- 2: Säule
- 3: Horizontalführung
- 4: Schwenklager
- 5: C-Bogenhalterung
- 6: C-Bogen
- 7: Röntgenstrahlenempfänger
- 8: Röntgenstrahlenquelle
- 9, 9', 9", 9"': Positionsmarke
- 10: Referenzmarke
- 11: Kodiermarke
- 12: Autoreflexionsmarke
- 13: LED-Marke
- 14: Lichtleitfasermarke
- 15: Antastmulde
- 16: Gehäuse
- 20, 20': Rolle
- Mi: Markenanordnung

## Patentansprüche

1. Röntgendiagnostikeinrichtung mit einem Steuerungsrechner und mehreren Einrichtungsteilen (1, 3, 5, 6, 7, 8) und mit von einem Lageerfassungssystem erfaßbaren Markenanordnungen (Mi) auf der Oberfläche der Gehäuse (16) der Einrichtungsteile (1, 3, 5, 6, 7, 8),
**dadurch gekennzeichnet, daß** jede Markenanordnung (Mi) jeweils durch einen 2D-Code repräsentiert wird, der einen Referenzpunkt auf der Oberfläche der Gehäuse (16) einschließt und daß die gesamte Oberfläche der Gehäuse (16) mit einer Vielzahl von Markenanordnungen (Mi) versehen ist, wobei der Code für jeden Referenzpunkt eindeutig mit der in einer look-up-table des Steuerungsrechners der Röntgendiagnostikeinrichtung hinterlegten Position des jeweiligen Referenzpunktes auf der Oberfläche verknüpft ist.

2. Röntgendiagnostikeinrichtung nach Anspruch 1,
**dadurch gekennzeichnet, daß** der 2D-Code der Markenanordnungen (Mi)auf einer ebenen Oberfläche angeordnet ist oder auf einer Zylindermantelfläche aufgerollt ist

3. Röntgendiagnostikeinrichtung nach einem der Ansprüche 1-2,
**dadurch gekennzeichnet, daß** die Marken (9, 10, 11) der Markenanordnungen (Mi) als Autoreflexionsmarken (12) auf der Oberfläche durch Kleben oder Aufdrucken ausgeführt sind.

4. Röntgendiagnostikeinrichtung nach einem der Ansprüche 1-2,
**dadurch gekennzeichnet, daß** die Marken (9, 10, 11) der Markenanordnungen (Mi) als LED-Marken (13) oder als Lichtleitfasermarken (14) in den Gehäusen (16) derart integriert sind, daß an den äußeren Oberflächen der Gehäuse (16) die Marken (9, 10, 11) als im wesentlichen punktförmige Lichtquellen erscheinen.

5. Röntgendiagnostikeinrichtung nach einem der Ansprüche 1-4,
**dadurch gekennzeichnet, daß** der 2D-Code ein Datamatrixcode ist.

6. Röntgendiagnostikeinrichtung nach einem der Ansprüche 1-5,
**dadurch gekennzeichnet, daß** die 2D-Codes der Markenanordnungen (Mi) ineinander verschachtelt sind und unterschiedliche Außenabmessungen aufweisen.

7. Verfahren zur Bestimmung der Lage von Einrichtungsteilen (1, 3, 5, 6, 7, 8) einer Röntgendiagnostikeinrichtung in den Koordinatensystemen eines Lageerfassungssystems und der Röntgendiagnostikeinrichtung unter Verwendung einer Röntgendiagnostikeinrichtung nach einem der Ansprüche 1-6,
**gekennzeichnet durch** folgende Verfahrensschritte:
• Aufnahme wenigstens eines Bildes der Röntgendiagnostikeinrichtung mit den Markenanordnungen (Mi) mit einer Kamera des Lageerfassungssystems
• Vornahme einer Vortransformation für jede auswertbare Markenanordnung (Mi) in einem Auswerterechner des Lageerfassungssystems mit dem Ziel, für jede Markenanordnung (Mi) jeweils ein Teilbild einer orthogonal aufgenommenen ebenen Markenanordnung (Mi) zu erhalten, wobei der Ort des Referenzpunktes im ursprünglichen Bild erhalten bleibt
• Automatische Erkennung der Codes in den Teilbildern und Zuordnung der Codes zu den Orten der Referenzpunkte im Koordinatensystem des Lageerfassungssystems
• Übergabe der Positionen der Referenzpunkte zusammen mit dem zugeordneten Code an den Steuerungsrechner der Röntgendiagnostikeinrichtung
• Berechnung der Positionen der Einrichtungsteile (1, 3, 5, 6, 7, 8) der Röntgendiagnostikeinrichtung in einem mit diesem verbundenen Koordinatensystem unter Verwendung der in der look-up-table hinterlegten Positionen der Referenzpunkte auf der Oberfläche der Einrichtungsteile (1, 3, 5, 6, 7, 8).
